# EUROPEAN PATENT SPECIFICATION

(11) **EP 0 529 008 B1**
(45) Date of publication and mention of the grant of the patent: **15.12.1999**
(21) Application number: 91920949.4
(22) Date of filing: 15.04.1991
(51) Int. Cl.: C12N 15/00

(54) **MODULATION OF GENE EXPRESSION THROUGH INTERFERENCE WITH RNA SECONDARY STRUCTURE**
MODULATION DER GENEXPRESSION DURCH EINGREIFEN IN DIE RNA SEKUNDÄRSTRUKTUR
PROCEDES DE MODULATION DE L'EXPRESSION DES GENES PAR L'INTERFERENCE DANS LA STRUCTURE SECONDAIRE DE L'ARN

(30) Priority: 04.05.1990 US 518929
(43) Date of publication of application: 03.03.1993
(60) Divisional application: 98203541.2
(73) Proprietor: ISIS PHARMACEUTICALS, INC., Carlsbad, CA 92008 (US)
(72) Inventor: ECKER, David, J., Carlsbad, CA 92009 (US)
(74) Representative: Hallybone, Huw George
(86) International application number: US9102558
(87) International publication number: WO9117246

(56) References cited:
- EP-A- 0 331 939
- EP-A- 0 386 563
- WO-A-91/04753
- US-A- 4 806 463
- NATURE vol. 331, 21 January 1988, MACMILLAN JOURNALS LTD.,LONDON, UK; pages 280 - 283 T. JACKS ET AL. 'Characterization of ribosomal frameshifting in HIV-1 gag-pol expression'
- SCIENCE vol. 246, 22 December 1989, AAAS,WASHINGTON, DC,US; pages 1625 - 1629 E.T.DAYTON ET AL. 'Functional analysis of CAR, the target sequence for the rev protein of HIV-1'
- Cell, Volume 59, issued 20 October 1989, B. BERKHOUT et al., "TAT Transactivates the Human Immunodeficiency Virus through a Nascent RNA Target", pages 273-282, see entire document.
- Nature, Volume 334, issued 14 July 1989, S. FENG et al., "HIV-1 tat transactivation requires the loop sequence within tar", pages 165-167, see entire document.
- Proceedings National Academy of Sciences, Volume 85, issued August 1988, J. GOODCHILD et al., "Inhibition of Human Immunodeficiency Virus Replication by Antisense Oligodeoxy Nucleotides", pages 5507-5511, see entire article, but particularly pages 5507, 5508 and 5510.

## Description

### Field of the Invention

This invention relates to the field of therapeutics, particularly the treatment of infections of the human immunodeficiency virus (HIV). It relates to the design, synthesis and application of oliqonucleotides and oligonucleotide analogs which inhibit the activity of HIV.

### Background of the Invention

This invention relates to materials and methods for modulating the activity of HIV RNA. The invention generally relates to the field of "antisense" compounds, compounds which are capable of specific hybridization with a nucleotide sequence of an RNA. In accordance with preferred embodiments, this invention is directed to methods for achieving therapeutic treatment of disease and regulating gene expression in experimental systems.

It is well known that most of the bodily states in mammals including infectious disease states, are effected by proteins. Such proteins, either acting directly or through their enzymatic functions, contribute in major proportion to many diseases in animals and man. Classical therapeutics has generally focused upon interactions with such proteins in efforts to moderate their disease causing or disease potentiating functions. Recently, however, attempts have been made to moderate the actual production of such proteins by interactions with molecules that direct their synthesis, intracellular RNA. By interfering with the production of proteins, it has been hoped to effect therapeutic results with maximum effect and minimal side effects. It is the general object of such therapeutic approaches to interfere with or otherwise modulate gene expression leading to undesired protein formation.

One method for inhibiting specific gene expression which has been adopted to some degree is the "antisense" approach, where oligonucleotide analogs complementary to a specific, target, messenger RNA, mRNA sequence are used. A number of workers have reported such attempts. Pertinent reviews include C.A. Stein & J.S. Cohen, *Cancer Research,* vol. 48, pp. 2659-2668 (1988); J. Walder, *Genes & Development,* vol. 2, pp. 502-504 (1988); C.J. Marcus-Sekura, *Anal. Biochemistry,* vol. 172, 289-295 (1988); G. Zon, *Journal of Protein Chemistry,* vol. 6, pp-131-145 (1987); G. Zon, *Pharmaceutical Research,* vol. 5, pp. 539-549 (1988); A. R. Van der Krol, J.N. Mol, & A.R. Stuitje, *BioTechniques,* vol. 6, pp. 958-973 (1988) and D.S. Loose-Mitchell, *TIPS,* vol. 9, pp. 45-47 (1988). Each of the foregoing provide background concerning general antisense theory and prior techniques.

Prior attempts to inhibit HIV by various antisense approaches have been made by a number of researchers. Zamecnik and coworkers have used phosphodiester oligonucleotides targeted to the reverse transcriptase primer site and to splice donor/acceptor sites P.C. Zamecnik, J. Goodchild, Y. Taguchi, P.S. Sarin, *Proc. Natl. Acad. Sci. USA* 83, 4143 (1986). Goodchild and coworkers have made phosphodiester compounds targeted to the initiation sites for translation, the cap site, the polyadenylation signal, the 5' repeat region and a site between the gag and pol genes. J. Goodchild, S. Agrawal, M.P. Civeira, P.S. Sarin, D. Sun, P.C. Zamecnik, *Proc. Natl. Acad. Sci. U. S. A.* 85, 5507 (1988). In the Goodchild study, the greatest activity was achieved by targeting the polyadenylation signal. Agrawal and coworkers have extended the studies of Goodchild by using chemically modified oligonucleotide analogs which were also targeted to the cap and splice donor/acceptor sites. S. Agrarwal, J. Goodchild, M.P. Civeira, A.H. Thornton, P.S. Sarin, P.C. Zamecnik, *Proc. Nat'l. Acad. Sci. USA* 85, 7079 (1988). A portion of one of these overlapped a portion of the HIV TAR region but was not found to have exemplary effect. Neither was this oligonucleotide analog designed to interfere with the HIV TAR region. Agrawal and coworkers have used oligonucleotide analogs targeted to the splice donor/acceptor site to inhibit HIV infection in early infected and chronically infected cells. S. Agrawal, T. Ikeuchi, D. Sun, P.S. Sarin, A. Konopka, J. Maizel, *Proc. Natl. Acad. Sci. U. S. A.* 86, 7790 (1989).

Sarin and coworkers have also used chemically modified oligonucleotide analogs targeted to the cap and splice donor/acceptor sites. P.S. Sarin, S. Agrawal, M.P. Civeira, J. Goodchild, T. Ikeuchi, P.C. Zamecnik, *Proc. Natl. Acad. Sci. U. S. A.* 85, 7448 (1988). Zaia and coworkers have also used an oligonucleotide analog targeted to a splice acceptor site to inhibit HIV. J.A. Zaia, J.J. Rossi, G.J. Murakawa, P.A. Spallone, D.A. Stephens, B.E. Kaplan, *J. Virol.* 62, 3914 (1988). Matsukura and coworkers have synthesized oligonucleotide analogs targeted to the initiation of translation of the rev gene mRNA. M. Matsukura, K. Shinozuka, G. Zon, et al, *Proc. Natl. Acad. Sci. USA* 84, 7706 (1987); R.L. Letsinger, G.R. Zhang, D.K. Sun, T. Ikeuchi, P.S. Sarin, *Proc. Natl. Acad. Sci. U. S. A.* 86, 6553 (1989). Mori and coworkers have used a different oligonucleotide analog targeted to the same region as Matsukura. K. Mori, C. Boiziau, C. Cazenave, et al., *Nucleic Acids Res.* 17, 8207 (1989). Shibahara and coworkers have used oligonucleotide analogs targeted to a splice acceptor site as well as the reverse transcriptase primer binding site. S. Shibahara, S. Mukai, H. Morisawa, H. Nakashima, S. Kobayashi, N. Yamamoto, *Nucl. Acids Res.* 17, 239 (1989). Letsinger and coworkers have synthesized and tested oligonucleotide analogs with conjugated cholesterol targeted to a splice site. K. Mori, C. Boiziau, C. Cazenave, et al., *Nucleic Acids Res.* 17, 8207 (1989). Stevenson and Iversen have conjugated polylysine to oligonucleotide analogs targeted to the splice donor and the 5'-end of the first exon of the tat gene. M. Stevenson, P.L. Iversen, *J. Gen. Virol.* 70, 2673 (1989).

These prior attempts at targeting HIV have largely focused on the nature of the chemical modification used in the oligonucleotide analog. Although each of the above publications have reported some degree of success in inhibiting some function of the virus, a general therapeutic scheme to target HIV and other retroviruses has not been found. Accordingly, there has been and continues to be a long-felt need for the design of oligonucleotides and oligonucleotide analogs which are capable of effective, therapeutic antisense use.

This long-felt need has not been satisfied by prior work in the field of antisense oligonucleotide therapy for HIV and other retroviruses and viruses. Others have failed to identify target sites in which antisense oligonucleotides or oligonucleotide analogs are therapeutically effective at reasonable rates of application.

### OBJECTS OF THE INVENTION

It is a principal object of the invention to provide therapies for human diseases, particularly the human immunodeficiency virus and other human retroviruses.

It is a further object of the invention to provide molecules, especially oligonucleotides and oligonucleotide analogs which perturb the structure of mRNA.

Yet another object of this invention is to modulate gene expression in cells.

A further object is to interfere with the secondary structure of RNAs through interaction of those structures with oligonucleotides or oligonucleotide analogs.

Another object is to effect such interference through formation of perturbed RNA secondary structures.

Another object is to effect such interference through formation of nucleotide triplexes.

These and other objects of this invention will become apparent from a review of the instant specification.

### SUMMARY OF THE INVENTION

A new paradigm for targeting antisense oligonucleotides to HIV and other retroviruses, viruses and other infectious agents has now been discovered. Prior attempts at antisense targeting to HIV have been focused on inhibition of the synthesis of some particular viral protein thought to be essential to the success of the infection. In the present invention, the same goal (inhibition of viral gene expression) is achieved, but greater, therapeutically significant activity is obtained by targeting particular sites on HIV RNA. In the present invention, target RNA structures which have important biological function have been found to be the key target sites. They are interfered with at the level of those structures. It has been determined that targeting these RNA structures is a key to effective antisense therapy with oligonucleotides and oligonucleotide analogs.

The invention provides an oligonucleotide or oligonucleotide analog which binds with a portion of RNA coded by at least part of the gag-pol element of HIV, said portion being capable of forming a secondary structure.

It is preferred that the oligonucleotide or oligonucleotide analog be capable of binding with at least about six subunits of the RNA subportion. It is more preferred that from eight to fifty units be capable of being bound, with from about 10 to about 20 subunits being even more preferred.

In accordance with preferred embodiments, the oligonucleotide of oligonucleotide analog is capable of forming a duplex structure with the subportion of RNA. Alternatively, and in accordance with certain preferred embodiments, the oligonucleotide or oligonucleotide analog can form a triplex structure with the selected portion of RNA. While the mechanism of the interaction is not known with certainty, it is possible that it may effect modulation of gene expression through a number of ways.

It is also intended that methods of treating animals suspected of having a disease characterized by expression of a gene coding for RNA having a secondary structure may also be provided. Thus, animals suspected of having the disease are contacted with oligonucleotides or oligonucleotide analogs which can bind with the secondary structure of the RNA implicated in the disease process. In particular, the present invention is believed to be suitable for the treatment of HIV infections in mammals, especially man. Thus, oligonucleotides or oligonucleotide analogs designed to interact with the gag-pol element of HIV are administered to animals, especially humans suspected of being infected with human immunodeficiency virus.

### BRIEF DESCRIPTION OF THE DRAWINGS

Figure 1 is a computer-predicted secondary structure of the gag-pol frame shift region and possible mechanisms of inhibition of frame shifting.

Figure 2 depicts antisense oligonucleotide interference with gag-pol frame shifting.

Figure 3 shows possible interference with gag-pol frame shifting through nucleotide triplex formation.

### DETAILED DESCRIPTION OF PREFERRED EMBODIMENTS

The biological function of RNA is mediated by its structure. mRNA is generally thought of as a linear molecule which contains the information for directing protein synthesis within the sequence of ribonucleotides. Recently, studies have revealed a number of secondary and tertiary structures in mRNA which are important for its function. I. Tinoco, Jr. P.W. Davis, C.C. Hardin, J.D. Puglisi, G.T. Walker, *Cold Spring Harb. Symp. Quant. Biol.* 52, 135 (1987). Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three dimensional structure.

Very little is known about the precise three dimensional structure of RNA. However, there have recently been a number of research efforts which have shown that RNA structures, including single stranded, secondary and tertiary structures, have important biological functions beyond simply encoding the information to make proteins in linear sequences. Some of these correlations have been discussed in the following publications: I. Tinoco, Jr., P.W. Davis, C.C. Hardin, J.D. Puglisi, G.T. Walker, *Cold Spring Harb. Symp. Quant. Biol.* 52, 135 (1987); O. Resnekov, M. Kessler, Y. Aloni, *J. Biol. Chem.* 264, 9953 (1989); C. Tuerk, P. Gauss, C. Thermes, et al., *Proc. Natl. Acad. Sci. U. S. A.* 85, 1364 (1988); and D.E. Larson, B.H. Sells, *Mol. Cell. Biochem.* 74, 5 (1987). Despite the fact that there is little precise structural information on RNA, a number of researchers have measured the binding energies of a large number of RNA duplex structures and have derived a set of rules which can be used to predict the secondary structure of RNA. J.A. Jaeger, D.H. Turner, M. Zuker, *Proc. Natl. Acad. Sci. USA* 86, 7706 (1989); D.H. Turner, N. Sugimoto, *Annu. Rev. Biophys. Biophys. Chem.* 17, 167 (1988). In conjunction with experimental data, these rules are useful in identification of RNA structural elements with important biological function.

It has been discovered to be possible to regulate the activity of RNA in cells by introducing oligonucleotides, or oligonucleotide analogs, which perturb or interfere with the secondary structure of natural RNA's. The oligonucleotides or oligonucleotide analogs interfere with the normal interaction between the RNA and the factors that bind to it. This method can be used to treat diseases, particularly HIV and other retroviruses. In accordance with the present invention, compositions which bind to biological RNA molecules with significant structural features of biological importance are provided. The present invention employs oligonucleotides and oligonucleotide analogs which bind to these structures. In the context of this invention, the term oligonucleotide refers to a plurality of joined nucleotide units formed from naturally-occurring bases and cyclofuranosyl groups joined by native phosphodiester bonds. This term effectively refers to naturally-occurring species or synthetic species formed from naturally-occurring subunits.

"Oligonucleotide analog," as that term is used in connection with this invention, refers to moieties which function similarly to oligonucleotides but which have non naturally-occurring portions. Thus, oligonucleotide analogs may have altered sugar moieties or inter-sugar linkages. Exemplary among these are the phosphorothioate and other sulfur containing species which are known for use in the art. They may also comprise altered base units or other modifications consistent with the spirit of this invention.

In accordance with certain preferred embodiments, at least some of the phosphodiester bonds of the oligonucleotide have been substituted with a structure which functions to enhance the ability of the compositions to penetrate into the region of cells where the RNA whose activity is to be modulated is located. It is preferred that such linkages be sulfur-containing. It is presently preferred that such substitutions comprise phosphorothioate bonds. Others such as alkyl phosphothioate bonds, N-alkyl phosphoramidates, phosphorodithioates, alkyl phosphonates, and short chain alkyl or cycloalkyl structures may also be useful. In accordance with other preferred embodiments, the phosphodiester bonds are substituted with structures which are, at once, substantially non-ionic and non-chiral. Persons of ordinary skill in the art will be able to select other linkages for use in the practice of the invention.

It is generally preferred for use in some embodiments of this invention that the 2' position of the linking sugar moieties in at least some of the subunits of the oligonucleotides or oligonucleotide analogs be substituted. Thus, 2' substituents such as OH, SH, F, OCH₃, OCN, OCHₙCH₃ where n is from 1 to about 20 and other substituents having similar properties may be useful in some embodiments.

Oligonucleotide analogs may also include species which include at least some modified base forms. Thus, purines and pyrimidines other than those normally found in nature may be so employed. Similarly, modifications on the cyclofuranose portions of the nucleotide subunits may also occur as long as the essential tenets of this invention are adhered to.

Such analogs are best described as being functionally interchangeable with natural oligonucleotides (or synthesized oligonucleotides along natural lines), but which have one or more differences from natural structure. All such analogs are comprehended by this invention so long as they function effectively to bind to selected portions of RNA having secondary structure of functional significance.

The oligonucleotides and oligonucleotide analogs in accordance with this invention preferably comprise from about 3 to about 100 subunits. It is preferred that such oligonucleotides and analogs comprise at least about 6 subunits with from about 8 to about 50 subunits being more preferred, and still more preferred to have from about 10 to about 20 subunits. As will be appreciated, a subunit is a base and sugar combination suitably bound to adjacent subunits through phosphodiester or other bonds.

The oligonucleotides and oligonucleotide analogs of this invention can be used in diagnostics, therapeutics and as research reagents and kits. For therapeutic use, the oligonucleotide or oligonucleotide analog is administered to an animal, especially a human, such as are suffering from a virus or retrovirus infection such as AIDS.

It is generally preferred to apply the therapeutic agent in accordance with this invention internally such as orally, intravenously or intramuscularly. Other forms of administration, such as transdermally, topically or intralesionally may also be useful. Inclusion in suppositories may also be useful. Use of the oligonucleotides and oligonucleotide analogs of this invention in prophylaxis is also likely to be useful. Use of pharmacologically acceptable carriers is also preferred for some embodiments. In accordance with the present invention, the oligonucleotides and oligonucleotide analogs which are useful in its performance are best described by the RNA whose secondary structure is to interfered with. Thus, it will be understood by persons of ordinary skill in the art that the oligonucleotides and analogs provided by this invention are those which are capable of binding with RNA having a secondary structure bearing a causal or mediative relationship to a diseased state. All such analogs are comprehended by this invention so long as they bind the target RNA structure at or adjacent to a secondary structure thereof.

A number of RNA secondary structures have recently been identified for which application of this invention will likely provide therapeutic utility. Others will also be useful as well. Some of these include the HIV TAR structures; S. Feng, E.C. Holland, *Nature* 334, 165 (1988), including the stem loops at nucleotide 1-59, and 60-104 according to the nucleotide sequence as described by Ratner L. Ratner L.; W. Haseltine, R. Patarca, K.J. Livak, B. Starcich, S.F. Josephs, *Nature* 313, 277 (1985); the boundary between the EGP/OMP regions of HIV, S. Le, J. Chen, M.J. Braun, M.A. Gonda, J.V. Maizel, *Nucl. Acids Res.* 16, 5153 (1988); the boundary between the TMP/env genes of HIV, S. Le, J. Chen, M.J. Braun, M.A. Gonda, J.V. Maizel, *Nucl. Acids Res.* 16, 5153 (1988); the HIV CAR structure, E.T. Dayton, D.M. Powell, A.I. Dayton, *Science* 246, 1625 (1989); the stem loop structure at the junction between the HIV gag and pol genes (nucleotides 1629-1674); the HIV CRS element; and the human iron responsive element (IRE) J.L. Casey, M.W. Hentze, D.M. Koeller, et al, *Science* 240, 924 (1988).

In addition, there are regions of RNA which are primarily thought of as single stranded areas which have been identified as sites for protein binding. For example, the sequence 5'-AUUUA-3' has been identified as a signal for a protein to bind which leads to degradation of RNA. J.S. Malter, Science 246, 664 (1989). The structure of this region in not known. However, that does not preclude the practice of this invention with this sequence. Additional RNA elements, with as yet unknown structures, can also be the subject of this invention.

It is not absolutely necessary to know the actual RNA structure in order to practice this invention, it is only necessary to know that a specific RNA sequence is recognized by an RNA binding element and that this interaction has important biological consequences. In this regard, the viral RNA sequences and structures which are recognized by the structural proteins of retroviruses for virion formation may be the subject of this invention as may many others. It is not intended that application of this invention be limited to presently known structures. Binding to any RNA structure which has an important biological function falls within the spirit and scope of this invention.

This disclosure provides several methods to interfere with the natural function of an RNA structural element and others will be apparent to persons skilled in the art. By using the rules of Watson-Crick hybridization and free energy predictions for hybridization of oligonucleotides or oligonucleotide analogs designed to be complementary to RNA which comprises secondary structures it has now been found that the oligonucleotides and oligonucleotide analogs will compete with internal RNA structures by forming stable heteroduplexes. The energy barriers to heteroduplex formation are overcome by designing oligonucleotides or oligonucleotide analogs to form a more stable heteroduplex than the internal RNA structure on the target RNA.

Kinetic considerations for strand invasion of an existing RNA duplex also enter into the oligonucleotide design. It is possible to disrupt an existing RNA secondary structure with an invading strand by designing the invading strand to have at least three, and preferably more bases complementary to regions to the target RNA which are not involved in base pairing. This provides a kinetic "foothold" for the invading strand to initiate the process of heteroduplex formation.

It is also disclosed that duplex RNA structure can be perturbed by binding to it by triple strand formation. In contrast to heteroduplex formation, where the RNA secondary structure is broken by an invading strand, triple strand formation generally preserves the existing RNA duplex hydrogen bonding pattern, but binds in a helical groove with additional hydrogen bonds. Triple strand formation is a phenomenon which has been known in a limited sense for some time. General reviews which describe triple strand formation with duplex DNA include; J.C Hanvey, M. Shimizu, R.D. Wells, *Proc. Natl. Acad. Sci. USA* 85, 6292 (1988); and S. Arnott, E. Selsing, *J. Mol. Biol.* 88, 509 (1974). Triple strand formation with RNA homopolymers has been previously described in S.L. Broitman, D.D. Im, J.R. Fresco, *Proc. Natl. Acad. Sci. USA* 84, 5120 (1987). It has not, however, previously been disclosed to inhibit the function of RNA by binding to duplex regions with oligonucleotides or oligonucleotide analogs by triple strand formation. It is now believed, however, that binding to regions of RNA secondary structure, such as in the stem regions of stem-loops, will perturb the interactions between the natural RNA and the factors which bind to it, thus modulating gene expression.

In accordance with the present invention, it will be understood that the term "to bind" as it refers to the interaction between an oligonucleotide or oligonucleotide analog and an RNA portion or subportion may have any of several, related meanings. Thus, the present invention comprehends binding of an oligonucleotide or analog with at least one of subportions forming a secondary structure of an RNA portion comprising them. It will be understood that the oligonucleotide or analog will bind with at least one of the subportions of the RNA portion in a Watson-Crick fashion so as to form, locally, a heteroduplex between the RNA subportion and the oligonucleotide or analog. This heteroduplex formation is believed to result in alteration of the secondary structure of the RNA portion. The exact mechanism and the result of this effect is not known with certainty, yet it is believed that the normal secondary structure of the RNA portion is gradually replaced by the binding of the oligonucleotide with one or more of the subportions of the RNA portion. Since the electronic and steric factors which attend the new heteroduplex are different from those of the natural-occurring RNA portion, the effectiveness and nature of the function to generate protein from the RNA is interfered with. The resulting formation of defective or missing protein manifests itself overall as a modulation in the expression of the gene coding for the RNA.

The present invention also comprehends the formation of triplexes with RNA portions having secondary structures. Once again, the precise nature of such triplexes is not fully understood however it is believed that suitably-constructed oligonucleotides or oligonucleotide analogs can so interact with portions of RNA having a secondary structure in some circumstances. The resulting triplex formation is believed to grossly interfere with translation of protein from the RNA thus leading to modulation of expression of the gene from which the RNA derives.

In accordance with the invention it is not necessary that the interaction of oligonucleotide or oligonucleotide analog with the RNA portion or subportion -- the binding of the two -- result in either non-formation or malformation of protein. It may be in some circumstances that interruption of some control or other function having a significant role in the gene expression protocol may be an effective means of modulating that expression. One example of this relates to the gag-pol locus in HIV. Thus, it is not necessary that protein translation be stopped or that defective proteins be produced. Rather, through interference of the gag-pol region it is believed possible to interfere with frame-shifting which is believed to lead to the preparation of fusion proteins of significant importance to the HIV organism.

In short, any interaction or binding of oligonucleotide or oligonucleotide analog with an RNA having a secondary structure is believed to have the potential for interference with RNA function and, hence, for modulation of the expression of the gene from which the RNA derives. It is likely that persons of ordinary skill in the art will find other means of interfering with RNA secondary structures other than those set forth with specificity herein. All such means are, however, contemplated by the present invention.

While a wide variety of oligonucleotides and oligonucleotide analogs are believed to be useful in practice of the present invention, it has been found to be preferred to design such oligonucleotides and analogs so as to bind with at least about six subunits of a subportion of an RNA portion having a secondary structure. In accordance with other preferred embodiments, oligonucleotides which combine with from about six to about 30 and even more preferably with about 10 to about 20 subunits are preferred.

Interference with the gag-pol element of HIV is preferred in accordance with the practice of this invention. In such case, it is expected that frame-shifting will be interfered with leading to the malformation or non-formation of essential proteins of the gag-pol family.

Therapeutics are particular objects of the present invention. Thus, presenting oligonucleotides and oligonucleotide analogs in accordance with the present invention in pharmaceutically acceptable carriers may be highly useful. It is desired to treat animals suspected of having diseases characterized by expression of the gag-pol element of HIV. Thus, animals suspected of having such diseases are contacted with oligonucleotides or oligonucleotide analogs which are designed to bind with a secondary structure of those RNAs. This is especially true for treatment of the disease AIDS.

Overall, it is preferred to administer to patients suspected of suffering from the foregoing disease states with amounts of oligonucleotide or analog, in either native form or suspended in a carrier medium in amounts and upon treatment schedules which are effective to reduce the symptomology of that disease. It is within the scale of a person's skill in the art to determine optimum dosages and treatment schedules for such treatment regimens.

### EXAMPLE . Inhibition of Frame Shifting: the HIV gap/pol Frameshift Region.

HIV and other retroviruses synthesize a protein which encodes a reverse transcriptase, pol, as part of a fusion with a structural protein known as gag. The virus also encodes a sequence-specific protease which cleaves between the gag and pol domains of the fusion protein to release free pol. In all retroviruses examined to date the genetic sequence of the gag-pol mRNA precludes direct translation of the mRNA into a gag-pol fusion protein. Either there is an in-frame termination codon between the gag and pol domains on the mRNA, or the gag and pol sequences are not in the same reading frame of the message. T. Jacks, M.D. Power, F.R. Masiarz, P.A. Luciw, P.J. Barr, H.E. Varmus, *Nature* 331, 280 (1988). In the case of HIV, the pol reading frame is -1 relative to gag. In order to express a fusion protein the ribosome "frame shifts" at the junction between the gag and pol regions on the mRNA and continues translation in the reading frame of pol until completion of synthesis of the fusion protein.

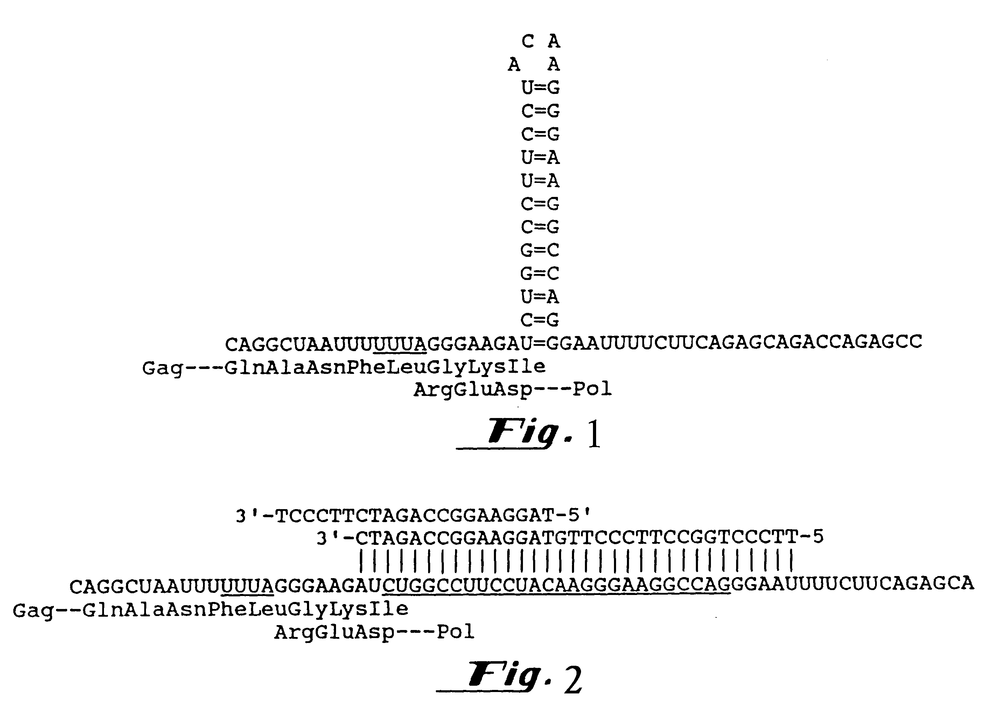

In HIV and other retroviruses, near the site of frame shifting, there is a potential for the formation of significant RNA secondary structure. A computer predicted structure for HIV-1 is illustrated in Figure 1. The potential formation of RNA secondary structures near the sites of ribosomal frame shifting exists in a number of viral gag-pol fusions. T. Jacks, K. Townsley, H. E. Varmus, J. Majors, *Proc. Natl. Acad. Sci. U. S.* A. 84, 4298 (1987); T. Jacks, M.D. Power, F.R. Masiarz, P.A. Luciw, P.J. Barr, H.E. Varmus, *Nature* 331, 280 (1988); and I. Brierley, P. Digard, S.C. Inglis, *Cell* 57, 537 (1989). It is now discovered that targeting the region between the HIV gag and pol genes with antisense oligonucleotides and oligonucleotide analogs is effective in modulating the expression of the gag-pol fusion protein. Figure 1 depicts the mRNA region of interest in the instant example, including the predicted stem-loop structure near the site of frameshifting and the predicted amino acid sequence of the gag and gag-pol fusion proteins near the frameshift site. Figure 2 depicts the sequences of two representative antisense oligonucleotides or oligonucleotide analogs which would be expected to modulate ribosomal frameshifting and translation of the target mRNA. By binding to this site by any of the following methods, it is possible to perturb the normal course of gene expression and as a result, inhibit the virus.

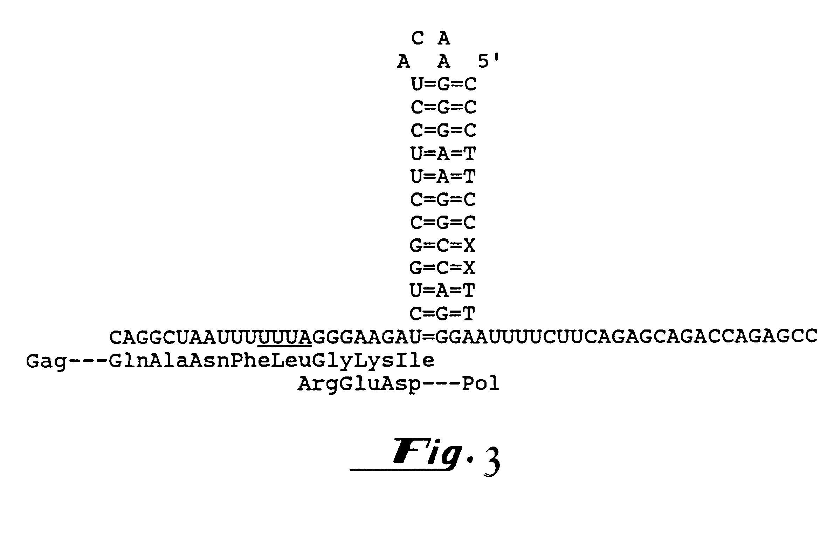

It is now discovered that compounds which specifically bind to the gag-pol frameshift region and interfere with translation and/or frameshifting are believed to have activity as therapeutic agents for retroviral infection. It is intended that all retroviruses which have RNA secondary structures at the gag-pol junctions fall within the spirit and scope of this invention. Different strains and types of retroviruses will have different gag-pol junctions with different secondary structures. This invention can be practiced on different strains or types of retroviruses by changing the sequence of the oligonucleotide or oligonucleotide analog to complement the structure of the alternative strain or type of retrovirus. Cells will be treated with the following oligonucleotides or oligonucleotide analogs: 
Sequence 5'--3'
TAGGAAGGCCAGATCTTCCCT
AAGAAAATTCCCTGGCCTTCCCTTGTAGGAAGGCCAG
TGCTCTGAAGAAAATTCCCT
TCTGAAGAAAATTCCCTGGC
 
GAAGAAAATTCCCTGGCCTT
CTGGCCTTCCCTTGTAGGAA
GCCTTCCCTTGTAGGAAGGC
TTCCCTTGTAGGAAGGCCAG
CCTTGTAGGAAGGCCAGATC
TGTAGGAAGGCCAGATCTTC
 It is also disclosed that the gag-pol RNA structure can be perturbed by binding to it by triple strand formation (see Figure 3. In contrast to heteroduplex formation, where the RNA secondary structure is broken by an invading strand, triple strand formation generally preserves the existing RNA duplex hydrogen bonding pattern, but binds in a helical groove with additional hydrogen bonds. Oligonucleotides of the following sequence will be tested: 
Sequence 5'--3'
CCCTTCCXXTT
CCTTCCXXT
GGGAAGGXXAG
GGAAGGXXA
 where X is any heterocyclic base containing a hydrogen bond acceptor. The assay for translation and ribosomal frame shifting has been previously described in T. Jacks, M.D. Power, F.R. Masiarz, P.A. Luciw, P.J. Barr, H.E. Varmus, *Nature* 331, 280 (1988). The assay will be performed as described with the addition of the above oligonucleotides and oligonucleotide analogs.

## Claims

1. An oligonucleotide or oligonucleotide analog which binds with a portion of RNA coded by at least part of the gag-pol element of HIV, said portion being capable of forming a secondary structure.

2. An oligonucleotide or oligonucleotide analog according to claim 1, comprising at least 6 subunits.

3. An oligonucleotide or oligonucleotide analog according to claim 2, comprising from 8 to 50 subunits.

4. An oligonucleotide or oligonucleotide analog according to claim 3, comprising from 10 to 50 subunits.

5. An oligonucleotide or oligonucleotide analog according to any preceding claim, capable of forming a duplex structure with said portion.

6. An oligonucleotide or oligonucleotide analog according to any preceding claim, capable of forming a triplex structure with said secondary structure.

7. An oligonucleotide or oligonucleotide analog according to any preceding claim in association with a pharmaceutically acceptable carrier.

## Patentansprüche

1. Oligonucleotid oder Oligonucleotid-Analogon, das sich mit einem RNA-Abschnitt verbindet, der durch mindestens einen Teil des gag-pol-Elements von HIV codiert wird, wobei der genannte Abschnitt eine Sekundärstruktur bilden kann.

2. Oligonucleotid oder Oligonucleotid-Analogon nach Anspruch 1, das mindestens 6 Untereinheiten umfaßt.

3. Oligonucleotid oder Oligonucleotid-Analogon nach Anspruch 2, das 8 bis 50 Untereinheiten umfaßt.

4. Oligonucleotid oder Oligonucleotid-Analogon nach Anspruch 3, das 10 bis 50 Untereinheiten umfaßt.

5. Oligonucleotid oder Oligonucleotid-Analogon nach irgendeinem vorhergehenden Anspruch, das mit dem genannten Abschnitt eine Duplex-Struktur bilden kann.

6. Oligonucleotid oder Oligonucleotid-Analogon nach irgendeinem vorhergehenden Anspruch, das eine Triplex-Struktur mit der genannten Sekundärstruktur bilden kann.

7. Oligonucleotid oder Oligonucleotid-Analogon nach irgendeinem vorhergehenden Anspruch in Kombination (Assoziation) mit einem pharmazeutisch akzeptablen Träger.

## Revendications

1. Oligonucléotide ou analogue d'oligonucléotide qui se fixe à une portion d'un ARN codé par au moins une partie de l'élément gag-pol du HIV, ladite portion étant capable de former une structure secondaire.

2. Oligonucléotide ou analogue d'oligonucléotide selon la revendication 1, comprenant au moins 6 sous-unités.

3. Oligonucléotide ou analogue d'oligonucléotide selon la revendication 2, comprenant de 8 à 50 sous-unités.

4. Oligonucléotide ou analogue d'oligonucléotide selon la revendication 3, comprenant de 10 à 50 sous-unités.

5. Oligonucléotide ou analogue d'oligonucléotide selon l'une quelconque des revendications précédentes, qui est capable de former une structure duplex avec ladite portion.

6. Oligonucléotide ou analogue d'oligonucléotide selon l'une quelconque des revendications précédentes, qui est capable de former une structure triple brin avec ladite structure secondaire.

7. Oligonucléotide ou analogue d'oligonucléotide selon l'une quelconque des revendications précédentes, en association avec un véhicule acceptable d'un point de vue pharmaceutique .
